# FASCICULE DE BREVET EUROPEEN

(11) **EP 0 605 351 B1**
(45) Date de publication et mention de la délivrance du brevet: **04.11.1998**
(21) Numéro de dépôt: 93490016.8
(22) Date de dépôt: 28.09.1993
(51) Int. Cl.: A61B 17/00

(54) **Instrument, destiné à l'extraction des tronçons veineux**
Instrument zur Extraktion eines Venenstücks
Instrument for the removal of a length of vein

(30) Priorité: 30.12.1992 FR 9216061
(43) Date de publication de la demande: 06.07.1994
(73) Titulaire: Duthoit, François Régis, F-59320 Escobecques (Nord) (FR)
(72) Inventeur: Duthoit, François Régis, F-59320 Escobecques (Nord) (FR)
(74) Mandataire: Duthoit, Michel

(56) Documents cités:
- EP-A- 0 115 751
- EP-A- 0 358 999
- CH-A- 632 659
- FR-A- 2 182 392
- FR-A- 2 649 309
- FR-A- 2 679 437
- GB-A- 2 082 459
- GB-A- 2 195 540
- US-A- 2 661 003
- US-A- 5 109 867
- SURGERY vol. 27 , Février 1950 , ST LOUIS pages 280 - 281 COLE AND HOLDEN 'A polyethylene rod vein stripper'

## Description

La présente invention a pour objet un instrument, destiné notamment à permettre l'extraction de tronçons veineux pathologiques tels que des varices.

Elle trouvera son application notamment dans le domaine de la fabrication de tels instruments ainsi que dans celui de son application dans le domaine chirurgical.

Actuellement, pour extraire des tronçons veineux, on connaît notamment des instruments, tels que celui décrit dans le document GB-A-2.195.540, constitués d'une tige terminée d'un anneau, orienté selon l'axe de la tige et présentant un diamètre supérieur à celui de la veine à traiter.

Ledit anneau est introduit autour de ladite veine puis est fait coulisser extérieurement, à l'aide de ladite tige, le long de celle-ci de manière à permettre le détachement des tissus qui l'entourent.

Un tel instrument autorise ainsi le prélèvement de tronçons veineux intacts. Toutefois, il s'agit d'interventions lourdes qui, de plus, ne présentent aucune utilité dans le cadre de l'extraction de varices puisque, dans ce cas, le tronçon veineux extrait n'a pas vocation a étre réutilisé.

Pour extraire des varices dans une veine sanguine variqueuse, par exemple de la saphène interne, on a recours à des techniques différentes telles que, notamment, l'éveinage ou 〈〈 stripping 〉〉.

Cette technique est utilisée lorsqu'un segment veineux est pathologique et irrécupérable. Lorsque ce segment est court, l'éveinage pourra être réalisé sous contrôle visuel après exposition complète du segment veineux à enlever.

Lorsque ce segment est long, l'éveinage devra être fait de manière différente pour une technique 〈〈 semi-aveugle 〉〉, qui consiste en l'arrachage de la plus grande partie du segment veineux sans contrôle visuel, celui-ci n'ayant été possible qu'au niveau de son extrémité proximale et distale par une courte incision permettant de reconnaître la veine, de la disséquer, de la cathétériser indifféremment de haut en bas ou de bas en haut, à l'aide d'instrument.

Pour cela, il est notamment connu une technique qualifiée 〈〈 d'exo-veinage 〉〉 qui consiste à extraire un tronçon de veine en arrachant celui-ci par l'intermédiaire d'un instrument qui comporte un fil flexible dont l'une des extrémités présente une forme de cône et dont l'autre des extrémités présente un organe en forme d'olive.

Au moyen d'un tel instrument, le chirurgien, lors de l'enlévement des varices dans une jambe, met à nu la veine en dessous de l'endroit de l'hernie et, introduit, l'extrémité en forme de cône de manière à faire dépasser, au niveau sensiblement de l'endroit de l'hernie à extraire, l'extrémité en forme de cône. Lorsque cette opération d'introduction est réalisée, la veine est accrochée sous l'olive qui possède un diamètre nettement plus grand que la veine. Le tronçon de veine est alors retiré par l'intermédiaire du chirurgien qui exerce une traction sur l'instrument en le faisant ressortir au niveau d'une seconde incision qui a été ménagée dans la jambe du patient.

De telles techniques, présentent en pratique, de nombreux inconvénients et notamment celui de nécessiter la réalisation de deux incisions nécessaires d'une part à l'introduction de l'instrument et d'autre part à son retrait.

Pour remédier à cet inconvénient, on a développé des instruments, tels que celui décrit dans le document FR-2.649.309, constitués de deux câbles reliés entre eux par un embout d'éveinage, l'un desdits câbles constituant des moyens de rappel dudit embout.

Après extraction du tronçon veineux, de tels instruments permettent ainsi de retirer l'embout par l'incision par laquelle il a été introduit et ne nécessitent donc qu'une seule incision large, le retrait de la veine à l'autre extrémité pouvant être réalisé par une incision de quelques millimètres seulement.

Toutefois, l'utilisation d'un second câble pour effectuer le retour de l'embout d'éveinage et la nécessité d'opérer sa liaison au premier câble grâce audit embout peuvent présenter des difficultés en raison des deux fonctions d'éveinage et de maintien assemblé remplies en conséquence simultanément par ledit embout. En effet, celles-ci peuvent se révéler incompatibles compte tenu des efforts de traction et de flexion auxquels ledit instrument est soumis.

Par ailleurs, les instruments d'intervention par exo-éveinage ne sont pas toujours adaptés pour permettre l'extraction de tronçons de veine relativement longs puisqu'il peut nécessiter la réalisation d'un nombre important d'incisions, ce qui peut être préjudiciable pour le patient.

Il faut également noter que l'utilisation de tels instruments chirurgicaux peut générer des traumatismes du nerf saphène qui résultent, en outre, des actions de traction exercées sur la veine par le chirurgien et/ou du nombre important, d'incisions nécessaires à de telles opérations.

Il est également connu, notamment du document US-2.661.003, une technique qualifiée de 〈〈 endo-veinage 〉〉 qui consiste à retirer la veine de la jambe en l'arrachant par l'intermédiaire d'un câble qui a été préalablement introduit à l'intérieur de celle-ci. En outre, par cette technique, le chirurgien extrait un tronçon veineux du tissu vivant qui le traverse en le faisant glisser progressivement contre ce dit tissu, et en exerçant une action pour que l'extrémité dudit tronçon se plisse longitudinalement.

Dans cette technique, on retourne la veine sur elle-même afin de diminuer le traumatisme péri-veineux. L'olive de l'exo-veinage de diamètre supérieur à la veine est ici remplacée, soit par un simple fil solidarisant le câble sur lequel elle est nouée durant l'intervention, à l'extrémité de la veine à invaginer, soit par un dispositif intermédiaire connectant deux câbles au moyen de leurs extrémités respectives en étant adaptées pour s'interconnecter notamment par cliquetage, et pour permettre ainsi l'éveinage, en va et vient, de la veine préalablement solidarisée au câble par une ligature solide, s'appuyant devant ou derrière cette connexion suivant le sens de la traction.

C'est ainsi que les instruments de phlébectonie par tringlage, actuellement connus, comprennent un corps longiligne souple tel que par exemple un câble dont les parties terminales présentant un renflement ou une excroissance permettant, en outre, la mise en place d'une poignée de traction et/ou d'un moyen d'arrêt de la veine à extraire.

Ces moyens d'arrêt sont,en outre, généralement constitués par un élément qui présente un diamètre au moins égal au diamètre de la partie terminale du tronçon veineux à extraire et, des formes et des moyens complémentaires permettant son arrimage et sa mise en place sur les excroissances dudit câble.

Or, en pratique, de tels moyens d'arrêt présentent de nombreux inconvénients et notamment, ils ne sont pas toujours d'un emploi aisé. Par ailleurs, en raison de leur diamètre au moins égal au diamètre extérieur du conduit, on observe, un raclement des tissus au cours de l'extraction de la veine à extraire ce qui a pour conséquence de générer des blessures et/ou des traumatismes physiologiques au tissu pouvant être préjudiciables pour le patient.

Par ailleurs, les différents nerfs qui se trouvent à côté de la veine saphène sont avec l'utilisation de tels instruments susceptibles d'être blessés ce qui rend douloureux de telles interventions pour le patient.

Toutefois, en pratique, bien que d'un emploi plus commode, de tels dispositifs n'apportent pas entière satisfaction car ils ne permettent pas d'assurer toujours un maintien efficace du tronçon veineux à extraire.

La présente invention a pour but de remédier aux inconvénients des instruments destinés notamment à permettre l'extraction de tronçons veineux en fournissant un instrument qui peut être utilisé pour mettre en oeuvre des techniques d'endo-éveingages et/ou d'exo-éveinages sans risque pour le patient.

Un autre but de l'instrument conforme à l'invention réside dans le fait qu'il est d'une conception simple et robuste, ce qui accroît ses capacités d'utilisation.

Un autre avantage de l'instrument conforme à l'invention réside dans le fait qu'il est réalisé à partir de matériaux présentant des caractéristiques appropriées conformes aux normes en vigueur pour de telles utilisations notamment dans le domaine chirurgical.

D'autres buts et avantages de la présente invention apparaîtront au cours de la description qui va suivre qui n'est donnée qu'à titre indicatif et qui n'a pas pour but de la limiter.

A cette fin, l'invention a pour objet un instrument, destiné notamment à permettre l'extraction de tronçons veineux tels que notamment des varices, comprenant :
a) deux câbles souples, flexibles et résistants, aptes à être introduits dans ladite veine à extraire, parmi lesquels :
   - le premier desdits deux câbles présente une longueur réduite correspondant sensiblement à au moins la longueur de ladite veine et un diamètre sensiblement inférieur à celui de ladite veine et comprend deux extrémités,
   - le second desdits deux câbles présente une longueur réduite correspondant sensiblement à au moins la longueur de ladite veine et un diamètre sensiblement inférieur à celui de ladite veine et comprend deux extrémités,
   - la première desdites deux extrémités dudit premier desdits deux câbles présente une pièce de liaison 〈〈 mâle 〉〉 et la première desdites deux extrémités dudit second desdits deux câbles présente une pièce de liaison 〈〈 femelle 〉〉, ladite pièce de liaison 〈〈 mâle 〉〉 et ladite pièce de liaison 〈〈 femelle 〉〉 étant complémentaires et constituant des moyens de solidarisation desdits deux câbles,
b) deux ogives supportées respectivement par la seconde desdites deux extrémités dudit premier desdits deux câbles et par la seconde desdites deux extrémités dudit second desdits deux câbles, la première et la seconde desdites deux ogives possédant un corps dans lequel est ménagé un orifice radial,
c) une olive qui présente un corps de forme sensiblement troncônique à l'intérieur duquel est ménagé un logement destiné à coopérer soit avec ladite pièce de liaison 〈〈 mâle, soit avec ladite pièce de liaison 〈〈 femelle 〉〉 de façon amovible.

La présente invention sera bien comprise à la lecture de la description suivante accompagnée des dessins en annexe parmi lesquels :
- la figure 1 est une vue de face qui illustre un câble qui supporte une pièce de liaison de l'instrument conforme à l'invention,
- la figure 2 est une vue de dessus qui illustre schématiquement un câble qui supporte une pièce de liaison de l'instrument similaire à celle de la figure 1,
- la figure 3 est une vue de face qui montre schématiquement un câble qui supporte une pièce de liaison, complémentaire de celle illustrée aux figures 1 et 2, conforme à l'invention,
- la figure 4 est une vue selon la ligne A-A de la figure 1,
- la figure 5 est une vue en coupe de la B-B de la figure 1,
- la figure 6 est une vue en coupe selon la ligne C-C de la figure 1,
- la figure 7 est une vue selon la flèche F₁ de la figure 1,
- la figure 8 est une vue selon la flèche F₂ de la figure 3,
- la figure 9 est une vue schématique en coupe partielle qui illustre une olive d'un instrument conforme à l'invention,
- la figure 10 est une vue de dessus de l'olive de l'instrument conforme à l'invention,
- la figure 11 est une vue schématique qui illustre une ogive d'un instrument conforme à l'invention,
- la figure 12 est une vue schématique qui illustre une ogive d'un instrument conforme à l'invention disposée à l'extrémité d'un câble de forme hélicoïdale d'un pas déterminé,

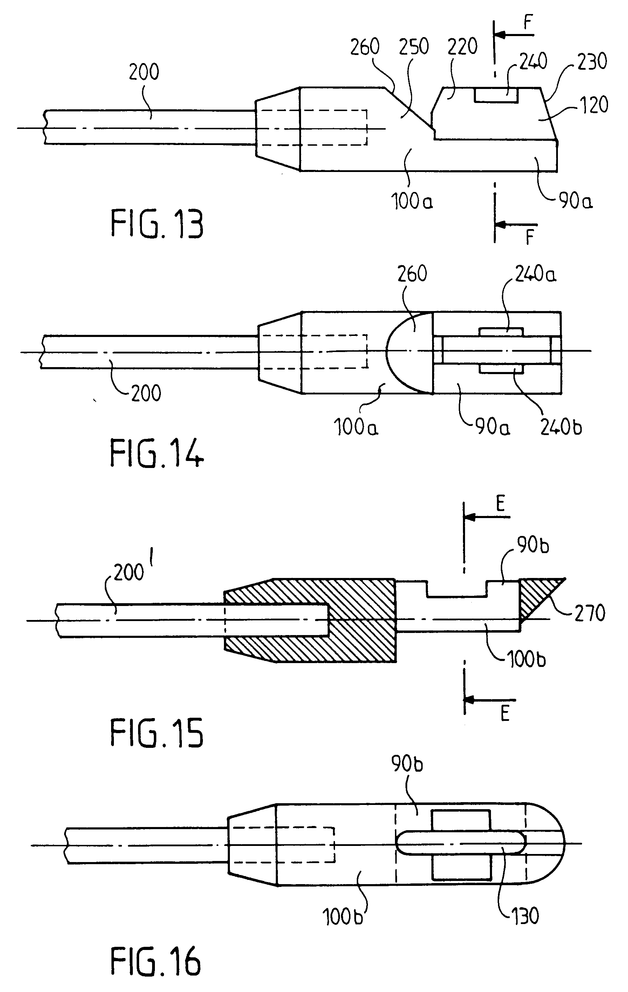

- la figure 13 est une vue de face schématique qui illustre un câble supportant une variante de réalisation d'une pièce de liaison de l'instrument conforme à l'invention,
- la figure 14 est une vue de dessus qui illustre schématiquement un câble qui supporte une pièce de liaison conforme à celle de la figure 13,
- la figure 15 est une vue de face schématique en coupe qui illustre un câble qui supporte une variante de réalisation d'une pièce de liaison complémentaire de celles illustrées aux figures 13 et 14,
- la figure 16 est une vue de dessus qui illustre une pièce similaire à celle de la figure 16,
- la figure 17 est une coupe selon la ligne E-E de la figure 14,
- la figure 18 est une vue en coupe selon la ligne F-F de la figure 15,
- la figure 19 est une vue schématique qui montre une variante de réalisation d'ogive conforme à l'invention,
- la figure 20 est une vue de dessus qui illustre une variante de réalisation d'une olive selon une conformation "bec de perroquet",
- la figure 21 est une vue schématique en coupe qui illustre la structure de l'extrémité du câble inséré dans la pièce de liaison,
- la figure 22 est une vue schématique en coupe partielle qui illustre une variante de réalisation des moyens d'accouplement des deux pièces complémentaires.

La présente invention a pour objet un instrument destiné notamment à permettre l'extraction de tronçons veineux tels que par exemple des varices. Elle trouvera son application dans le domaine de la fabrication et de l'utilisation de tels instruments à caractère médical et/ou chirurgical.

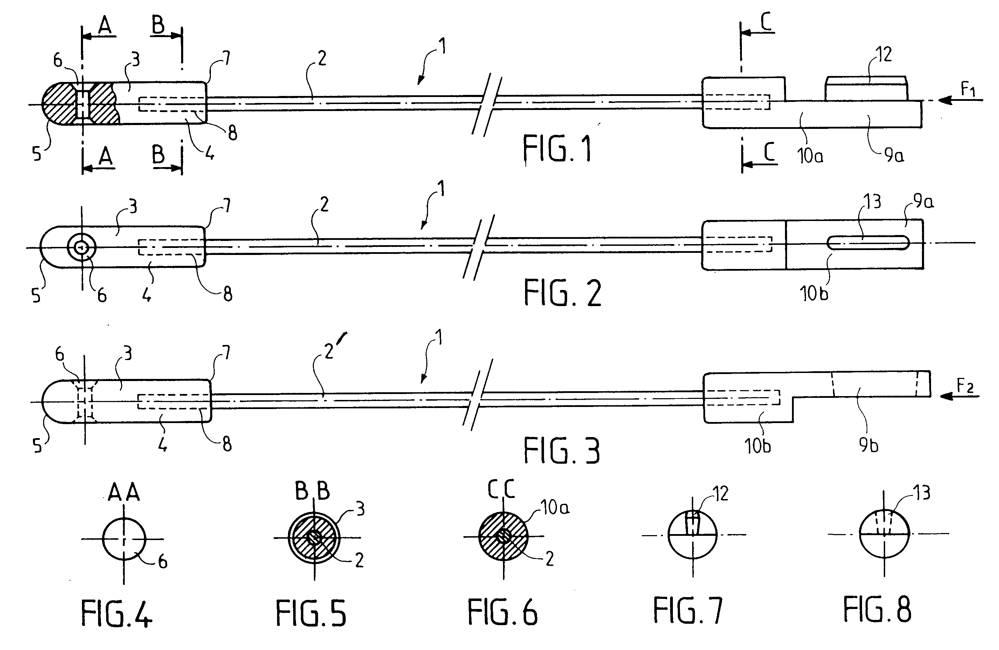

En se référant plus particulièrement aux figures 1, 2 et 3, on voit que l'instrument 1 conforme à l'invention comprend deux câbles 2, 2', 200, 200' flexibles, souples, et résistants réalisés par exemple en un matériau plastique ou, en une matière métallique qui présente des propriétés et des caractéristiques compatibles avec des normes d'utilisation notamment d'hygiène en milieu hospitalier et/ou médical.

Notamment, on a obtenu de bons résultats à partir d'un câble 2 réalisé dans du polyester ou Polyéthylène téréphtalate "PET" ou bien en polyamide.

Ce câble 2 présente par ailleurs un diamètre compatible avec celui des tronçons veineux à extraire, et notamment un diamètre extérieur légèrement inférieur au diamètre intérieur des dits tronçons veineux.

La longueur de ce câble 2 est variable en fonction des applications et il peut être par exemple compris entre 1 m et 1,20 m.

Une ogive 3 est prévue sur le câble. Elle présente dans une forme de réalisation un corps 4 ayant une forme généralement cylindrique arrondie à son extrémité 5 opposée à celle de son raccordement au câble 2. Elle est en outre réalisée en une matière plastique ou en une matière métallique qui présente des propriétés, et des caractéristiques appropriées pour l'usage médical.

Le corps 4 de l'ogive présente également sensiblement en regard de l'extrémité arrondie 5, un orifice radial 6 le traversant de part en part tel qu'il est plus particulièrement illustré à la figure 4.

L'extrémité 7 du corps 4 de l'ogive 3, en regard de l'extrémité arrondie 5, comporte un alésage axial 8 s'étendant sensiblement sur au moins une partie de la longueur du dit corps 4 afin de permettre l'introduction et le maintien de l'une des extrémités du câble 2 en vue d'autoriser l'assujettissement de l'olive 3 sur celui-ci.

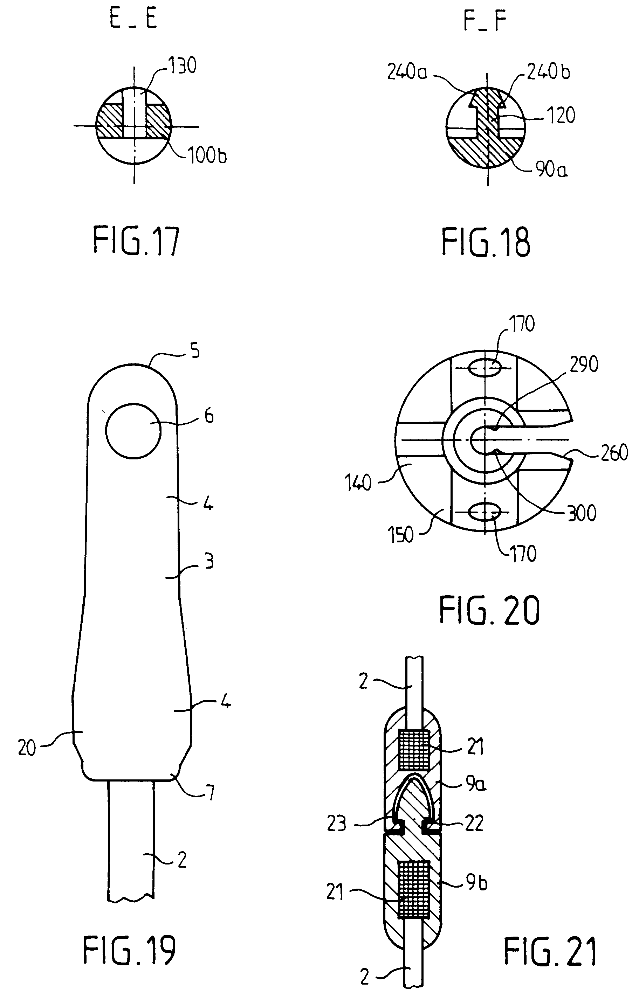

Dans une variante de réalisation illustrée à la figure 19, le corps 4 de l'ogive 3 présente à son extrémité 7, un épaulement radial 20 dont l'épaisseur et l'inclinaison sont adaptées. Cet épaulement 20 permet de faciliter l'utilisation du dispositif conforme à l'invention dans le cas de veines sinueuses ou lors de franchissement de valvules, en permettant notamment de mieux les franchir.

Cette ogive 3 est, constituée par un corps 4 qui présente un diamètre par exemple de 3,5 mm de manière à permettre le cathétérisme de la veine saphène lors de son introduction dans la dite veine en vue de permettre l'extraction du tronçon veineux que l'on désire enlever.

En outre, l'orifice radial 6 permet l'accrochage et le maintien de la veine pour assurer un retrait de celle-ci selon la technique de l'endo-eveinage en autorisant son maintien et son accrochage par l'intermédiaire d'un fil, par exemple en polyester, sur un fil de rappel non représenté, sur lequel le chirurgien exerce une traction pour assurer le retrait du dit tronçon veineux que l'on désire extraire dans l'axe du câble.

Il est également à remarquer que malgré la souplesse de la veine à extraire, celle-ci peut se rompre sous l'action des contraintes de traction générées par le décollement de sa paroi à l'encontre d'une veine collatérale. Dans ce cas, il est alors possible, conformément à l'invention, de réaliser un arrachement du dit tronçon veineux en inversant le sens de traction et notamment en reprenant les opérations d'extraction depuis son extrémité indemme c'est-à-dire en ligaturant cette dernière par l'intermédiaire du fil en polyester dans l'orifice 6.

Ainsi, grâce à l'invention, on accroît les possibilités d'utilisation de l'instrument sans nécessiter de la part du chirurgien des opérations longues et complexes susceptibles de générer des troubles du corps du patient.

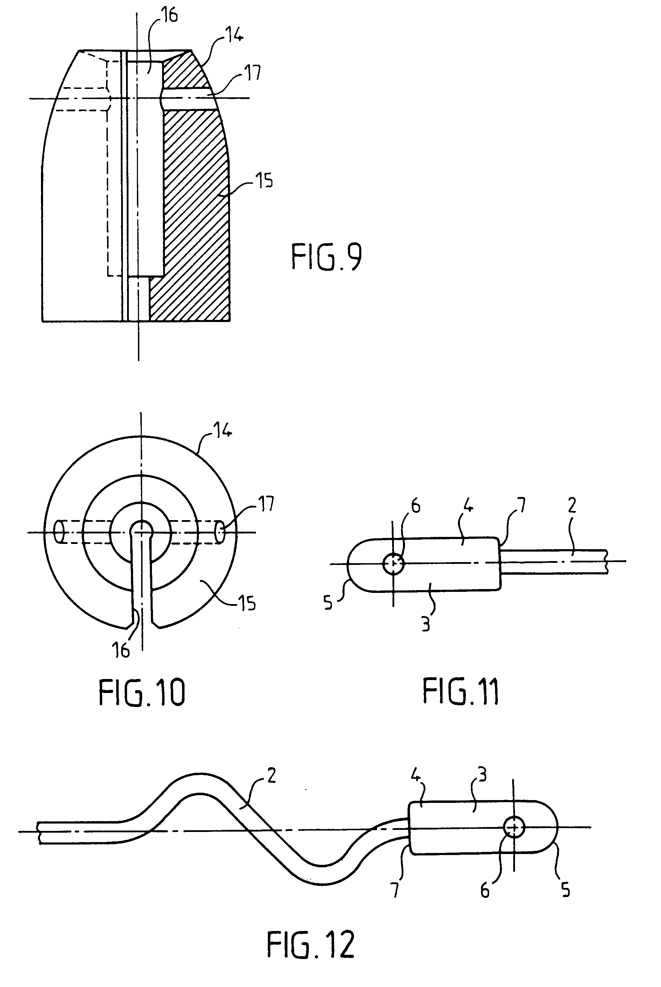

Par ailleurs, dans une forme de réalisation particulière de l'invention, comme cela est plus particulièrement illustré à la figure 12, le câble 2 présente au voisinage de l'extrémité 7 du corps 4 de l'olive 3 une forme hélicoïdale d'un pas déterminé de manière à permettre et à faciliter son introduction et sa mise en place à l'intérieur du tronçon veineux que l'on désire extraire.

Bien entendu, en fonction des applications et, notamment du diamètre intérieur de la veine à extraire, le pas de ce tronçon de câble hélicoïdal est variable ainsi que le nombre de spires et/ou d'ondulations qu'ils comportent.

L'instrument 1 conforme à l'invention comporte également des pièces de liaison complémentaires respectivement mâle et femelle 9a-9b, réalisées en un matériau plastique ou en un matériau métallique, qui présentent des propriétés et des caractéristiques qui permettent son utilisation dans des applications médicales et/ou chirurgicales.

Ces pièces de liaison complémentaires 9a-9b présente respectivement un corps 10a-10b dans lequel est ménagé un orifice 11 axial fixé sensiblement sur une partie de la longueur de celui-ci et à l'intérieur duquel est placée l'une des extrémités du câble 2 de manière à permettre le maintien et l'assujettissement de cette pièce de liaison sur le dit câble 2.

En ce qui concerne le maintien du câble 2, dans ces pièces de liaison 9-9b, on prévoit un écrasement de son extrémité 21 comme plus particulièrement montré à la figure 21 afin de lui conférer une structure maillée par des stries longitudinales et transversales sensiblement perpendiculaires entre elles.

Dans une forme de réalisation illustrée aux figures 1 et 2, le corps 10a de la pièce de liaison 9a comporte un téton 12 faisant saillie perpendiculairement qui est destiné à coopérer en position montée, comme il sera décrit ultérieurement, avec un orifice 13 de forme complémentaire à celui du téton 12 ménagé dans le corps 10b de la pièce de liaison 9b complémentaire assujettie à l'extrémité d'un câble 2, illustré à la figure 3.

Ainsi, selon l'invention, l'on peut réaliser, à partir de l'instrument 1, une association d'un câble 2 tel que celui qui est plus particulièrement illustré aux figures 1 et 2 avec un câble tel que celui qui est plus particulièrement illustré à la figure 3. Cette association est obtenue par la coopération du téton 12 du corps 10a de la pièce de liaison 9a qui vient se placer à l'intérieur de l'orifice 13 ménagé dans le corps 10b de la pièce de liaison 9b complémentaire.

Grâce à cette association de deux câbles, on peut réaliser une connexion qui permet au chirurgien de réaliser l'extraction d'un tronçon de veine par la technique d'endo-eveinage sans nécessiter l'utilisation d'un fil de rappel ce qui facilite son intervention tout en limitant les risques pour le patient.

Par ailleurs, dans le cas où la technique d'endo-eveinage ne pourrait pas être mise en oeuvre, la connexion de deux câbles, l'instrument 1 conforme à l'invention permet de mettre en oeuvre également la technique d'exo-eveinage, dans le même sens de traction que celui mis en oeuvre pour la technique d'exo-eveinage, ce qui accroît ses capacités d'utilisation tout en permettant au praticien de réaliser dans de bonnes conditions l'opération d'extraction de la veine.

Cette possibilité d'utilisation de l'instrument 1 conforme à l'invention pour mettre en oeuvre la technique d'exo-eveinage est appréciable notamment dans le cas où il y a une rupture, par la technique d'endo-eveinage, sur une veine colatérale, avec possibilité de récupérer l'olive au moyen d'un fil de rappel, par l'incision où elle a été introduite.

Dans une variante de réalisation, on prévoit une pièce de liaison dont le corps 100a comporte un téton 120 présentant des pans coupés 220-230 ainsi que deux nervures longitudinales 240a-240b s'étendant de part et d'autre du corps en faisant saillie sensiblement dans sa partie médiane. Par ailleurs, l'extrémité 250 du corps 100a de la pièce de liaison 90a présente également un pan coupé 260 selon une inclinaison adaptée par exemple de 58 °.

Le corps 100b de la pièce de liaison 90b comporte un orifice oblong 130 de forme complémentaire du téton 120, et qui est destiné à le recevoir afin d'assurer l'association des deux câbles. Par ailleurs, le corps 100b de la pièce de liaison 90b présente des pans coupés 270 selon une inclinaison complémentaire de celle du pan 260.

Dans cette forme de réalisation, l'association des deux câbles s'effectue par encliquetage du téton 120 dans l'orifice 130 et cette opération est notamment facilitée par l'intermédiaire des nervures 240a-240b qui permettent par ailleurs également d'assurer un meilleur maintien relatif des deux pièces complémentaires respectivement mâle et femelle 90a-90b. Par ailleurs, ces pans coupés 260-270 facilitent également le retrait du téton de l'orifice lorsque l'on désire dissocier les deux câbles 200 200'.

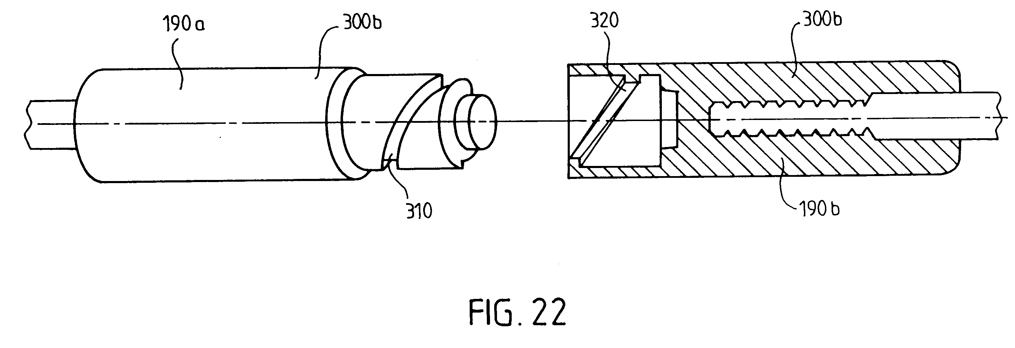

Dans une variante de réalisation illustrée à la figure 22, le corps 300a de la pièce 190a à l'une de ses extrémités présente un filetage 310 d'un pas déterminé. Dans ce cas, le corps 300b de la pièce complémentaire 190b de liaison comporte à l'une de ses extrémités un taraudage 320 d'un pas déterminé complémentaire de celui du filetage 310 de manière à permettre la solidarisation de ces deux pièces 190a-190b lorsque l'on assemble les deux câbles.

Bien entendu, on pourra également prévoir de solidariser les deux pièces complémentaires par tous moyens appropriés qui assurent une retenue et un maintien adaptés compatibles avec l'utilisation du dispositif.

C'est ainsi que, comme illustré à la figure 21, le corps 10b de la pièce 9b comporte un embout 22 de forme adaptée en fonction des applictaions, telle que par exemple sous la forme d'un tronc de cône arrondi apte à coopérer par encliquetage avec un orifice 23 de forme complémentaire ménagé dans le corps 10a de la pièce 9a.

L'instrument 1 de l'invention comporte également une olive 14 de forme sensiblement troncônique réalisée dans un matériau plastique ou dans une matière métallique appropriée pour de telles applications médicales. Cette olive 14 présente un corps 15 à l'intérieur duquel est ménagé un logement 16 permettant sa coopération et sa mise en place sur l'une des pièces de liaison 9a-9b.

Il faut également noter que la mise en place, du corps 15 de l'olive 14 se fait de façon amovible sur une des pièces de liaison 9a-9b de manière simple et rapide ce qui facilite sa mise en place et son retrait et donc l'utilisation de l'instrument 1.

Par ailleurs, le corps 15 de l'olive 14 comporte également un orifice radial 17 afin de pouvoir y fixer un fil de rappel autorisant notamment la sortie séparée de la veine à extraire du dispositif ce qui permet en outre d'éviter les traumatismes dans la zone du nerf saphène et permettant ainsi une cicatrice réduite.

Dans une forme de réalisation illustrée à la figure 20, le corps 150 de l'olive 140 présente un logement axial 280 pour permettre la mise en place et le maintien du corps d'une olive et d'une fraction de tronçon de câble. Plus précisément, ce logement qualifié de "bec de perroquet" comporte deux lignes en saillie parallèles 290-300 disposées en vis-à-vis afin d'assurer un meilleur maintien du câble à l'intérieur. Une butée non représentée est également prévue à l'une des extrémités du logement pour retenir l'olive lorsque celle-ci est placée à l'intérieur de celui-ci, ainsi qu'un logement 170 radial pour y fixer un fil de rappel.

Bien entendu, d'autres mises en oeuvre de la présente invention, auraient pu être envisagées sans pour autant sortir du cadre de celle-ci.

## Revendications

1. Instrument (1), destiné notamment à permettre l'extraction de tronçons veineux tels que, par exemple, des varices, comprenant :
a) deux câbles (2, 2', 200, 200'), souples, flexibles et résistants, aptes à être introduits dans ladite veine à extraire, parmi lesquels :
- le premier desdits deux câbles présente une longueur réduite correspondant sensiblement à au moins la longueur de ladite veine et un diamètre sensiblement inférieur à celui de ladite veine et comprend deux extrémités,
- le second desdits deux câbles présente une longueur réduite correspondant sensiblement à au moins la longueur de ladite veine et un diamètre sensiblement inférieur à celui de ladite veine et comprend deux extrémités,
- la première desdites deux extrémités dudit premier desdits deux câbles présente une pièce de liaison 〈〈 mâle 〉〉 (9a) et la première desdites deux extrémités dudit second desdits deux câbles présente une pièce de liaison 〈〈 femelle 〉〉 (9b), ladite pièce de liaison 〈〈 mâle 〉〉 et ladite pièce de liaison 〈〈 femelle 〉〉 étant complémentaires et constituant des moyens de solidarisation desdits deux câbles (2, 2', 200, 200'),
b) deux ogives (3) supportées respectivement par la seconde desdites deux extrémités dudit premier desdits deux câbles (2) et par la seconde desdites deux extrémités dudit second desdits deux câbles (2), la première et la seconde desdites deux ogives possédant un corps (4) dans lequel est ménagé un orifice radial (6),
c) une olive (14) qui présente un corps (15) de forme sensiblement troncônique à l'intérieur duquel est ménagé un logement (16) destiné à coopérer soit avec ladite pièce de liaison 〈〈 mâle 〉〉 (9a), soit avec ladite pièce de liaison 〈〈 femelle 〉〉 (9b).

2. Instrument selon la revendication 1, caractérisé en ce que la pièce de liaison 〈〈 mâle 〉〉 (9a) comporte un corps (10a) qui présente des moyens de solidarisation constitués par un téton (12) faisant saillie perpendiculairement.

3. Instrument selon la revendication 2, caractérisé en ce que la pièce de liaison complémentaire 〈〈 femelle 〉〉 (9b) comporte un corps (10b) qui présente des moyens de solidarisation constitués par un orifice axial (13) de forme complémentaire au téton (12).

4. Instrument selon la revendication 1, caractérisé en ce que le corps (15, 150) de l'olive (14, 140) comporte un orifice radial (17, 170).

5. Instrument selon la revendication 1, caractérisé en ce que l'une des extrémités du câble (2) en regard de l'ogive (3) présente une conformation hélicoïdal d'un pas déterminé.

6. Instrument selon la revendication 1, caractérisé en ce que l'ogive (3) a un corps (4) sensiblement cylindrique dont l'une des extrémités (5) est arrondie.

7. Instrument selon la revendication 1, caractérisé en ce qu'il est réalisé en des matériaux compatibles avec une utilisation dans le domaine médical ou chirurgical.

8. Instrument selon la revendication 3, caractérisé en ce que le corps (100a) de la pièce de liaison (90a) comporte des moyens de solidarisation constitués par un téton (120) dont le corps présente des pans coupés (220-230) ainsi que deux nervures longitudinales (240a-240b) s'étendant sensiblement dans sa partie médiane.

9. Instrument selon la revendication 8, caractérisé en ce que le corps (100a) de la pièce (90a) présente un pan coupé (260).

10. Instrument selon la revendication 9, caractérisé en ce que le corps (100b) de la pièce de liaison (90b) comporte un orifice oblong (130) de forme complémentaire au téton (120) et un pan coupé (270) selon une inclinaison complémentaire du pan coupé (260) de la pièce (90a).

11. Instrument selon la revendication 1, caractérisé en ce qu'il comporte une olive (140) dont le corps (150) présente un logement axial (280) qui comporte deux lignes en saillie (290-300) parallèles disposées en vis-a-vis.

12. Instrument selon la revendication 2, caractérisé en ce que le corps (300a) de la pièce de liaison (190a) comporte à l'une de ses extrémités un filetage (310) apte à coopérer avec un taraudage (320) ménagé à l'une des extrémités de la pièce de liaison complémentaire (190b).

13. Instrument selon la revendication 2, caractérisé en ce que le câble (2) présente un écrasement de l'une de ses extrémités (21) constitué par une structure maillée par des stries longitudinales et transversales sensiblement perpendiculaires entre elles.

## Claims

1. Instrument (1), designed, in particular, to permit the extraction of vein sections such as, for example, varices, including :
a) two supple, flexible, strong wires (2, 2', 200, 200'), capable of being introduced into the said vein to be extracted, wherein :
- the first of the said two wires is of a short length corresponding substantially to at least the length of the said vein, has a diameter substantially smaller than that of the said vein and comprises two ends ;
- the second of the said two wires is of a short length corresponding substantially to at least the length of the said vein, has a diameter substantially smaller than that of the said vein and comprises two ends ;
- the first of the said two ends of the said first one of the said two wires has a 〈〈 male 〉〉 connecting piece (9a) and the first of the said two ends of the said second one of the said two wires has a 〈〈 female 〉〉 connecting piece (9b), the said 〈〈 male 〉〉 connecting piece and the said 〈〈 female 〉〉 connecting piece being complementary and constituting means for rendering the said two wires (2, 2', 200, 200') integral;
b) two bullet shaped members (3) supported respectively by the second of the said two ends of the said first one of the said two wires (2) and by the second of the said two ends of the said second one of the said two wires (2), the first and the second of the said two heads having a body (4) in which is provided a radial orifice (6) ;
c) a olive shaped member (14) which has a body (15) of a substantially frustoconical shape inside which is provided a housing (16) designed to co-operate either with the said 〈〈 male 〉〉 connecting piece (9a) or with the said 〈〈 female 〉〉 connecting piece (9b).

2. Instrument according to claim 1, characterised in that the 〈〈 male 〉〉 connecting piece (9a) comprises a body (10a) which has joining means constituted by a perpendicularly projecting nipple (12).

3. Instrument according to claim 2, characterised in that the complementary 〈〈 female 〉〉 connecting piece (9b) comprises a body (10b) which has joining means constituted by an axial orifice (13) the shape of which mates with the nipple (12).

4. Instrument according to claim 1, characterised in that the body (15, 150) of the bead (14, 140) comprises a radial orifice (17, 170).

5. Instrument according to claim 1, characterised in that one of hte ends of the wire (2) facing the head (3) has a helical conformation of a given pitch.

6. Instrument according to claim 1, characterised in that the head (3) has a substantially cylindrical body (4) one of the ends (5) of which is rounded.

7. Instrument according to claim 1, characterised in that it is made of materials compatible with use in the medical or surgical field.

8. Instrument according to claim 3, characterised in that the body (100a) of the connecting piece (90a) comprises joining means constituted by a nipple (120) the body of which has cant faces (220-230), as well as two longitudinal ribs (240a-240b) extending substantially in its median portion.

9. Instrument according to claim 8, characterised in that the body (100a) of the connecting piece (90a) has a cant face (260).

10. Instrument according to claim 9, characterised in that the body (100b) of the connecting piece (90b) comprises an oblong orifice (130) of a form mating with the nipple (120) and a cant face (270) the inclination of which is complementary to the cant face (260) of the connecting piece (90a).

11. Instrument according to claim 1, characterised in that it comprises a bead (140) the body (150) of which has an axial housing (280) which comprises two parallel projecting lines (290-300) arranged facing each other.

12. Instrument according to claim 2, characterised in that the body (300a) of the connecting piece (190a) comprises, at one of its ends, a threaded portion (310) capable of cooperating with a tapped portion (320) provided at one of the ends of the complementary connecting piece (190b).

13. Instrument according to claim 2, characterised in that the wire (2) has a flattened portion on one of its ends (21) constituted by a structure criss-crossed by longitudinal and transverse striations substantially perpendicular to one another.

## Patentansprüche

1. Instrument (1) zum Ermöglichen der Entfernung von Aderbschnitten, wie z.B. Krampfadern, umfassende:
a) zwei weiche, biegsame und starke Kabel (2, 2'; 200, 200'), die geeignet sind in die genannte zu entfernende Ader eingeführt zu werden, von denen:
- das erste der genannten zwei Kabel eine kurze, im wesentlichen wenigstens der Länge der genannten Ader entspechende Länge und einen Durchmesser, der im wesentlichen kleiner ist als dernjenige der genannten Ader, aufweist und zwei Enden umfaßt,
- das zweite der genannten zwei Kabel eine kurze, im wesentlichen wenigstens der Länge der genannten Ader entspechende Länge und einen Durchmesser, der im wesentlichen kleiner ist als dernjenige der genannten Ader, aufweist und zwei Enden umfaßt,
- das erste der genannten zwei Enden des genannten ersten der genannten zwei Kabel einen "männlichen" Verbindungsteil (9a) und das erste der genannten zwei Enden des genannten zweiten der genannten zwei Kabel einen "weiblichen" Verbindungsteil (9b) aufweist, wobei der genannte "männliche" Verbindungsteil und der genannte "weibliche" Verbindungsteil einander ergänzend sind und Verbindungsmittel für die genannten zwei Kabel (2, 2'; 200, 200') bilden,
b) zwei respektive vom zweiten der genannten zwei Enden des genannten ersten der genannten zwei Kabel (2) und vom zweiten der genannten zwei Enden des genannten zweiten der genannten zwei Kabel (2) getragene Spitzbogenköpfe, wobei der erste und der zweite der genannten zwei Spitzbogenköpfe einen Körper (4) aufweisen, in dem eine Radialöffnung (6) vorgesehen ist,
c) eine Olive (14), die einen im wesentlichen kegelstumpfartigen Körper (15) aufweist, in dem ein Hohlraum (16) zum Zusammenwirken entweder mit dem genannten "männlichen" Verbindungsteil (9a) oder mit dem genannten "weiblichen" Verbindungsteil (9b) vorgesehen ist.

2. Instrument nach Anspruch 1, dadurch gekennzeichnet, daß der "männliche" Verbindungsteil (9a) einen Körper (10a) umfaßt, der aus einer senkrecht vorstehenden Warze (12) bestehende Verbindungsmittel aufweist.

3. Instrument nach Anspruch 2, dadurch gekennzeichnet, daß der ergänzende "weibliche" Verbindungsteil (9b) einen Körper (10b) umfaßt, der aus einer Axialöffnung (13) einer die Warze (2) ergänzenden Form bestehende Verbindungsmittel aufweist.

4. Instrument nach Anspruch 1, dadurch gekennzeichnet, daß der Körper (15, 150) der Olive (14, 140) eine Radialöffnung (17, 170) umfaßt.

5. Instrument nach Anspruch 1, dadurch gekennzeichnet, daß eines der Enden des Kabels (2) gegenüber dem Spitzbogenkopf (3) eine Spiralform mit einem bestimmten Gang aufweist.

6. Instrument nach Anspruch 1, dadurch gekennzeichnet, daß der Spitzbogenkopf (3) einen im wesentlichen zylindrischen Körper (4) hat, dessen eines Ende (5) abgerundet ist.

7. Instrument nach Anspruch 1, dadurch gekennzeichnet, daß es aus einem mit einer Verwendung auf dem ärztlichen oder chirurgischen Gebiet vereinbaren Material hergestellt ist.

8. Instrument nach Anspruch 3, dadurch gekennzeichnet, daß der Körper (100a) des Verbindungsteils (90a) Verbindungsmittel umfaßt, die aus einer Warze (120) bestehen, deren Körper abgestumpfte Ecken (220-230) sowie zwei sich im wesentlichen in dessen Mittelteil ersteckende Längrippen (240a-240b) aufweist.

9. Instrument nach Anspruch 8, dadurch gekennzeichnet, daß der Körper (100a) des Teils (90a) eine abgestumpfte Ecke (260) aufweist.

10. Instrument nach Anspruch 9, dadurch gekennzeichnet, daß der Körper (100b) des Verbindungsteils (90b) ein Langloch (130) einer die Warze (120) ergänzenden Form und eine gemäß einer die abgestumpfte Ecke (260) des Teils (90a) ergänzenden Neigung abgestumpfte Ecke (270) umfaßt.

11. Instrument nach Anspruch 1, dadurch gekennzeichnet, daß es eine Olive (140) umfaßt, deren Körper (150) einen Axialhohlraum (280) aufweist, der zwei gegenüber einander angeordnete, gleichlaufende, hervorstehende Linien (290-300) umfaßt.

12. Instrument nach Anspruch 2, dadurch gekennzeichnet, daß der Körper (300a) des Verbindungsteils (190a) an einem seiner Enden ein Außengewinde (310) umfaßt, das geeignet ist, mit einem an einem der Enden des ergänzenden Verbindungsteils (190b) vorgesehenen Innengewinde (320) zusammenzuwirken.

13. Instrument nach Anspruch 2, dadurch gekennzeichnet, daß das Kabel (2) eine Zerquetschung eines seiner Enden (21) aufweist, die aus einer mittels im wesentlichen senkrecht zu einander verlaufender Längs- und Querriffelungen vernetzten Struktur besteht.
